# EUROPEAN PATENT SPECIFICATION

(11) **EP 2 705 362 B1**
(45) Date of publication and mention of the grant of the patent: **10.02.2016**
(21) Application number: 12720852.8
(22) Date of filing: 04.05.2012
(51) Int. Cl.: G01N 33/50

(54) **USE OF SS'-DIMETHYL-GLIOTOXIN AS DIAGNOSTIC MARKER OF PATHOLOGIES CAUSED BY FUNGI PRODUCING GLIOTOXIN OR DERIVATIVES OF GLIOTOXIN**
VERWENDUNG VON SS'-DIMETHYL-GLIOTOXIN ALS DIAGNOSTISCHER MARKER VON KRANKHEITEN, DIE VON GLIOTOXIN- ODER GLIOTOXINDERIVATEN- PRODUZIERENDEN PILZEN VERURSACHT WERDEN
uTILISATION DE SS'-DIMÉTHYL-GLIOTOXINE EN TANT QUE MARQUEUR DE DIAGNOSTIC DE PATHOLOGIES PROVOQUÉES PAR DES CHAMPIGNONS PRODUISANT DE LA GLIOTOXINE OU LEURS DÉRIVÉS

(30) Priority: 04.05.2011 ES 201130711
(43) Date of publication of application: 12.03.2014
(73) Proprietor: Universidad De Zaragoza, 50009 Zaragoza (ES); Fundacion Agencia Aragonesa para la Investigación Y el Desarrollo (ARAID), 50018 Zaragoza (ES); Consejo Superior de Investigaciones Científicas (CSIC), 28006 Madrid (ES)
(72) Inventor: PARDO JIMENO, Julián, 50018 Zaragoza (ES); GÁLVEZ BUERBA, Eva M., 28006 Madrid (ES); DOMINGO REGIDOR, M. Pilar, E-28006 Madrid (ES)
(74) Representative: Pons Ariño, Angel
(86) International application number: PCT/EP2012/058247
(87) International publication number: WO 2012/150339

(56) References cited:
- WO-A2-2008/075395
- GARDINER DONALD M ET AL: "The epipolythiodioxopiperazine (ETP) class of fungal toxins: distribution, mode of action, functions and biosynthesis", MICROBIOLOGY, SOCIETY FOR GENERAL MICROBIOLOGY, READING, GB, vol. 151, 1 April 2005 (2005-04-01), pages 1021-1032, XP009160260, ISSN: 1350-0872
- PURI ALKA ET AL: "Development of an HPTLC-based diagnostic method for invasive aspergillosis", BIOMETRICAL JOURNAL,, vol. 24, no. 8, 1 August 2010 (2010-08-01) , pages 887-892, XP009160252, ISSN: 1099-0801
- DOMINGO MARIA P ET AL: "Bis(methyl)gliotoxin proves to be a more stable and reliable marker for invasive aspergillosis than gliotoxin and suitable for use in diagnosis.", DIAGNOSTIC MICROBIOLOGY AND INFECTIOUS DISEASE MAY 2012 LNKD- PUBMED:22480566, vol. 73, no. 1, April 2012 (2012-04), pages 57-64, XP9160254, ISSN: 1879-0070
- LATGÉ JP: "Aspergillus fumigatus and Aspergillosis", CLIN MICROBIOL REV, vol. 12, no. (2), 1999, pages 310-350,
- MASCHMEYER, G. ET AL: "Invasive Aspergillosis", DRUGS, vol. 67, no. 11, 2007, pages 1567-1601,
- AGARWAL, R.: "Allergic Bronchopulmonary Aspergillosis", CHEST, vol. 135, no. 3, 2009, pages 805-826,

## Description

The present invention relates to the use of a derivative of gliotoxin as marker for the diagnosis of pathologies caused by gliotoxin-producing fungi, **p**referably of the genus *Aspergillus.* Therefore, the invention could be included in the field of medicine.

### STATE OF THE ART

Fungi (moulds and yeasts) produce a great variety of compounds called secondary metabolites (antibiotics, alkaloids, etc.), which are not necessary for growth, but can improve survival in competition with other organisms. Gliotoxin (GT) is one of said metabolites and is produced by fungi and yeasts of different species, such as species of *Aspergillus, Penicillium and Trichoderma.* GT is a compound of the family of epipolythiodioxopiperazines (ETP), with immunomodulating activity, which is characterized by the presence of a disulfide bond in the piperazine ring. The presence of the disulfide bond in GT is essential for its biological activity. The blocking of the disulfide bond by methylation inactivates the toxin which results in the formation of SS'-dimethylgliotoxin (bmGT). bmGT is biologically inactive (Mullbacher A et al. 1986 Mol Immunol 23(2)231-235).

Opportunistic infections caused by fungi have been described, among which we can highlight infections by fungi of the genus *Aspergillus* and *Penicillium,* both in animals and humans, the so-called aspergillosis and penicilliosis. In clinical practice, an increase in infections caused by these fungi has been seen in immunodepressed patients, for example, in patients with human immunodeficiency virus (HIV), patients with cancer or patients with treatments or diseases that affect the immune system (Maschmeyer G et al. 2007 Drugs 67(11):1567-1601; Sternberg S. 1994. Science 266 (5191):1632-1634).

Among the different categories of aspergillosis, the one known as invasive aspergillosis (or invasive pulmonary aspergillosis, IA) stands out; it is an opportunistic infection caused by moulds of the genus *Aspergillus* (principally *Aspergillus fumigatus*), which has a high mortality rate in immunodepressed patients (higher than 80% mortality). This genus has the greatest presence within environmental fungal pathogens and a great increase has been observed in the number of infections caused by this genus in the last years. This fact is largely due to the development of increasingly effective therapies, but also more immunosuppressant, against cancer or during transplants. Furthermore, it has been suggested that climate change associated to a variation in both the temperature and humidity of the ecological niches where *Aspergillus* inhabits, may favour the increase in the number of spores of this mould in the environment and, therefore, the risk of this type of infection. Despite the increase in the number of hospital infections and deceased patients, there are still no markers available that allow a fast and reliable diagnosis of this infection. The current diagnostic techniques are not very specific, which means that many patients who are not infected by *Aspergillus* are treated with expensive antifungal drugs.

*Aspergillus fumigatus* (*A. fumigatus*) is an opportunistic human pathogen with a high presence in the air which causes a considerable number of nosocomial infections, especially in immunodepressed patients (Tekaia F et al. 2005 Curr Opin Microbiol 8(4):385-392). A. *fumigatus* is responsible for more than 90% of invasive aspergillosis (IA) in patients infected by HIV, with tuberculosis, with cancer or who undergo organ transplants, and may result in a mortality rate over 80%. One of the main problems related to IA is that the disease is only conclusively identified at a late stage, when it is already too serious and treatments have become ineffective.

Within the different toxins produced by A. *fumigatus*, GT has captured a special interest since it exercises an immunosuppressant activity both *in vitro* and *in vivo*, as it interferes with various immunological responses and has cytotoxic activity. Furthermore, it has been demonstrated that GT is capable of inducing apoptosis in different types of cells, including macrophages, fibroblasts and liver cells among others. Recently, GT was identified as a virulence factor of A. *fumigatus*, in an IA mouse model after corticosteroid-induced immunodepression. GT is produced in the initial phase of IA and its immunosuppressant activity contributes to aggravating the pathological effects observed in the infected host. GT has been detected in samples of patients with aspergillosis (Lewis RE et al. 2005 Infect Immun 73(1):635-637) and it has been postulated as an early diagnosis marker for the detection and prognosis of IA. However, GT is quickly eliminated from culture supernatants during *in vitro* incubation with a variety of cell types (Waring P et al. 1994 Toxicon 32(4):491-504). In the event that the biologically active GT can also be quickly converted *in vivo*, it could be removed from the body fluids by the adjacent tissues and by the circulating cells and, therefore, it may not be detectable therein, meaning that GT is not a good marker of infection by *Aspergillus.*

One of the main drawbacks of invasive aspergillosis lies in the detection methods, since normally when said infection is detected, the mould has already caused irreversible damages, hence its high degree of mortality. The current diagnosis techniques have a very low specificity, are expensive and late, so that when the infection is detected, treatments are no longer effective. The aspergillosis diagnosis methods used to date are, for example, microscopic observation of a sample, culture, galactomannan detection, (1-3)β-D-glucan detection or fungal deoxyribonucleic acid (DNA) detection (Rubio MC et al. 2007. Enferm Infecc Microbiol Clin 25 Suplm 3:45-51). At present, the diagnosis of aspergillosis is only classified as possible or probable and a diagnosis can only be confirmed after death or using highly invasive methods such as pulmonary biopsy.

In consequence, there is the need for a fast, accurate and sensitive tool for the detection of this type of disease, with the aim of improving the early diagnosis, prognosis and monitoring of aspergillosis in human beings for the preparation of a personalized treatment of the patient.

### DESCRIPTION OF THE INVENTION

The present invention discloses a sensitive, fast and precise *in vitro* method for the diagnosis, prognosis and/or monitoring of pathologies caused by fungi producing gliotoxin or derivatives of gliotoxin, characterized by the detection and quantification of SS'-dimethyl-gliotoxin (bmGT) in a biological sample. The biological sample may be, among others, sample of serum, plasma, blood or bronchoalveolar lavage. The present invention shows the usefulness of bmGT as marker for the diagnosis, prognosis and/or monitoring of pathologies caused by a gliotoxin-producing fungus or its derivatives in a subject.

The method of the invention is based on the detection and quantification of bmGT by separation with chromatography, preferably high performance thin layer chromatography (HPTLC) and detection using ultraviolet spectroscopy (UV). With this method it is possible to detect both bmGT and GT and differentiate between both. The present invention demonstrates the use of bmGT to detect GT-producing fungi or their derivatives. Results are shown that indicate that bmGT is a more sensitive marker than GT in the diagnosis of aspergillosis. The invention shows results of bmGT detection in biological samples of immunodepressed patients with a high reliability, sensitivity and specificity in the detection.

The present invention also relates to the use of bmGT as a new diagnostic marker of pathologies caused by gliotoxin-producing fungi or their derivatives which offers greater sensitivity than current diagnostic methods of fungal disease, which makes this a very useful method for the early diagnosis of said diseases. In relation to aspergillosis, the invention provides a method useful for the early detection of the disease, confirmation thereof and in the prognosis and/or monitoring of the patient. Furthermore, the detection of this marker using the method disclosed in the present invention is faster and more economical than other methods, which entails a much lower financial cost than the current markers, which would favour a reduction in the costs of public healthcare systems, as well as its implementation in underprivileged areas. The use of bmGT as aspergillosis marker, would be a great advance in reducing the high mortality rates of this infection.

From that described herein, a first aspect of the invention relates to an *in vitro* method for the diagnosis, prognosis and/or monitoring of pathologies caused by gliotoxin-producing fungi or their derivatives characterized by the detection of SS'-dimethyl-gliotoxin in the biological sample of a subject.

The term "*in vitro*" refers to the method of the invention being performed outside the body of the subject.

The term "diagnosis", as used in the present invention, refers to the capacity of detecting the gliotoxin-producing fungus or its derivatives, as well as discriminating between samples from patients and healthy individuals.

The term "prognosis" in the present invention refers to the capacity of detecting patients or asymptomatic subjects which have a high probability of suffering infection due to gliotoxin-producing fungi or their derivatives, even when said pathology is not present at the time of diagnosis. The term "prognosis" also relates to the capacity of detecting subjects previously diagnosed with infection by gliotoxin-producing fungi or their derivatives, with a high probability of suffering a deterioration or improvement of the disease.

The term "monitoring" refers to the evaluation of the infection in patients, for example, patients who have received treatments with antifungal drugs.

"Pathology" is understood, in the present invention, as the disease caused by the infection or presence of a gliotoxin-producing fungus or its derivatives. This pathology may be localized in various parts of the body of a subject, for example in the respiratory tract, for example, in the lungs.

"Gliotoxin-producing fungi", in the present invention, are those eukaryotic organisms of the *Fungi* kingdom that produce gliotoxin as secondary metabolite. The term "gliotoxin" (GT) relates to a compound of the family of epipolythiodioxopiperazines which are characterized in that they have the presence of a disulfide bond in the piperazine ring and which has immunomodulating activity, also called "active gliotoxin" (Gardiner DM, 2005. Microbiology 151 (Pt 4):1021-1032; Waring P, 1988, Medicinal Research Reviews, 4: 499-524; Gordon W. J. Chem. Soc., Perkin Trans. 1, 1980, 119-121). In the present invention the term "derivatives" relates to the compounds that are selected from the list comprising: gliotoxin E, gliotoxin G, S-methylgliotoxin or SS'-dimethyl-gliotoxin.

SS'-dimethyl-gliotoxin, also called dimethyl-gliotoxin, bis(dithio)bis(methylthio)gliotoxin, bis(methylthio)gliotoxin (bmGT) or according to the IUPAC classification (3R,5aS,6S,10aR)-6-hydroxy-3-(hydroxymethyl)-2-methyl-3,10a-bis(methylsulfonyl)-6,10-dihydro-5aH-pyrazino[1,2-a]indol-1,4-dione, (Waring P, 1988, Medicinal Research Reviews, 4: 499-524) (Gordon W. J. Chem. Soc., Perkin Trans. 1, 1980, 119-121) is a dimethylated derivative of gliotoxin lacking the disulfide bond and which does not have biological activity.

The term "biological sample" includes, but is not limited to, tissues and/or biological fluids of an individual, obtained using any method known by a person skilled in the art which serves for said purpose. The biological sample could be, for example, but is not limited to, a tissue or a sample of fluid, like blood, plasma, serum, bronchoalveolar lavage, lymph or ascitic fluid. Likewise, the biological sample may be, for example, but is not limited to, fresh, frozen, fixed or fixed and embedded in paraffin.

The term "subject" is understood to be that individual susceptible to infection by a GT-producing fungus or its derivatives.

A preferred embodiment of the first aspect of the invention relates to a method where the detection is carried out by the following steps:
a) separation of SS'-dimethyl-gliotoxin in the sample, by chromatography;
b) detection and/or quantification of the compound separated in (a) by ultraviolet spectroscopy.

Hereinafter, we will refer to this preferred embodiment as the "first method of the invention".

Chromatography is understood to be the analytical technique used for separation of a complex mixture through the use of a stationary phase and a mobile phase. This separation is based on the differentiation in behaviour of the compounds in the mixture both due to its physicochemical properties and its capacity of interacting with specific molecules. This chromatography can be performed both in columns and in plates and the separation can be carried out using solutions with specific properties that make it possible to separate said compounds. In the present invention it can be carried out using liquid chromatography ("high-performance liquid chromatography", HPLC) or thin layer chromatography ("high-performance thin layer chromatography", HPTLC). It can also be carried out using affinity chromatography using molecules that specifically bind to SS'-dimethyl-gliotoxin. Another even more preferred embodiment of the first aspect of the invention relates to a method where the detection of step (a) is performed using thin layer chromatography (HPTLC), hereinafter "second method of the invention".

HPTLC is understood to be the chromatography that is carried out using a solid support formed by an adsorbent material (stationary phase) which could be, without excluding, other silica gels, aluminium oxide, siliceous earth, celluloses or polyamides which are fixed on a solid support that could be, without excluding others, glass, plastic or metals such as aluminium. In this case the samples that have been extracted from the aforementioned fluids with a solvent or mixture of organic solvents such as dichloromethane, chloroform, methanol or others, are applied on the plate and after evaporation of the solvent, they are separated using a solution composed of mixtures of pure compounds such as, for example, n-heptane (C7), tetrahydrofuran (THF) and acetonitrile (ACN), for example, in proportion 40:58:2 (v: v: v). The separation is carried out in accordance with the different polarities of the compounds of the mixture.

A preferred embodiment of the first and second methods of the invention relates to the method where the detection of step (b) is performed between 220 and 400 nm. An even more preferred embodiment relates to the method where the detection of step (b) is performed at 280nm.

For the quantification of the compound separated in step (a) of the methods of the invention, it can be compared with a control sample.

The term "control samples" as understood in the present invention refers, for example, but is not limited to, a sample of known concentration of the compound which is to be quantified or it can also refer to a sample obtained from an individual infected with a GT-producing fungus or its derivatives. This type of control sample is a positive control sample.

The determination of the diagnosis of infection by GT-producing fungi or their derivatives may be determined by the detection of a limit value from which the sample is considered to be positive and, therefore, can be diagnosed as infected. The prognosis and the monitoring of the pathologies caused by these fungi can be performed by comparing with a control sample and if the difference between the value obtained for the sample and the value of the control sample is statistically significant, determine the favourable or unfavourable prognosis or the efficacy of an antifungal treatment.

In the present invention "statistically significant difference" refers to the existence of statistical differences between the compared values, the statistical probability being at least greater than 0.05 (p>0.05) or greater than 0.005 (p>0.005) and obtaining this according to the statistical test applicable in each case.

Another preferred embodiment of the first aspect of the invention relates to the method where the fungus is a mould or a yeast, preferably is a mould, more preferably is a mould of the genus *Aspergillus or Penicillium.* An even more preferred embodiment relates to a mould of the genus *Aspergillus,* and an even more preferred embodiment to the method where the mould is *Aspergillus fumigatus.*

The term "mould" is understood to be a fungus equipped with filamentous and branched mycelium wherefrom protrudes a rod which ends in a spherical sporangium, by way of a head, which lives in an organic medium rich in nutritional materials. In the present invention mould is understood as that fungus which causes a mycosis, an infection, in a subject, preferably it relates to the mould of the genus *Aspergillus,* which produces aspergillosis, or of the genus *Penicillium,* which produces penicilliosis.

The term *Aspergillus* in the present invention can refer to any species of the genus *Aspergillus,* for example *A. caesiellus, A. candidus, A. clavatus, A. defletus, A. flavus,* A. *fumigatus*, *A. glaucus, A. nidulans, A. niger, A. ochraceus, A. oryzae, A. parasiticus, A. penicliloides, A. restrictus,* A. *sojae, A. sydowi, A. terreus, A. ustus,* or *A. versicolor.* Preferably, it refers to *A. fumigatus.*

Another preferred embodiment of the first aspect of the invention relates to the method where the pathology is aspergillosis.

"Aspergillosis" is understood as a disease caused by a fungus of the genus *Aspergillus.* Aspergillosis may produce invasive pulmonary aspergillosis, recurrent chronic invasive pulmonary aspergillosis, allergic bronchopulmonary aspergillosis, aspergillomas, invasive tracheobronchitis, sinusitis, meningitis, malignant otitis externa, endocarditis, myocarditis or osteomyelitis, among others. The disease generally occurs due to inhalation of the fungus spores.

The present invention also relates to the method where the pathology is penicilliosis. "Penicilliosis" is understood as that disease caused by a fungus of the genus *Penicillium,* for example by *P. marneffei.*

Another preferred embodiment of the first aspect of the invention relates to a method where the subject is human. Another preferred embodiment relates to the method where the subject is an immunodepressed subject.

"Immunodepressed", also called immunosuppressed, immunocompromised or immunodeficient, is that subject with a reduced immune response, where said immunosuppression may be due to different causes, such as, for example, chemotherapy or chemical immunodepression, due to an infection by a pathogen such as fungi, virus or bacteria, due to a genetic factor, such as mutation or deletion of a specific gene, or it may be a newborn subject.

The biological sample wherein bmGT is detected *in vitro* may be selected from several biological samples. Hence, a preferred embodiment of the first aspect of the invention relates to a method where the biological sample is selected from serum, plasma, blood or bronchoalveolar lavage. The obtainment and processing of the biological samples can be performed using the means known by any person skilled in the art.

The detection of bmGT could be combined with the detection of other biological components or metabolites of fungi. Therefore, another preferred embodiment of the first aspect of the invention relates to a method where it further comprises the detection of biological components or metabolites of fungi, where said components or metabolites are selected from the list comprising: galactomannan, (1-3)beta-D-glucan, DNA, antigens, gliotoxin, gliotoxin E, Gliotoxin G, S-methylgliotoxin, helvolic acid, sporidesmin, sterigmatocystin, aflatoxins, ochratoxins, fumonisins or any of their combinations. It is also possible to use any other metabolite or biological component of fungi not present in the list which, in combination with bmGT, is useful in the method of the first aspect of the invention.

A more preferred embodiment relates to the method which further comprises the detection of gliotoxin. An even more preferred embodiment relates to the method where the detection of gliotoxin is performed using the following steps:
a) separation of gliotoxin in the sample, by chromatography;
b) detection and/or quantification of the compound separated in (a) by UV spectroscopy, preferably it is performed between 220-400 nm, more preferably is performed at 280 and 367 nm.

Furthermore, the methods of the invention could also be combined with other methods such as ELISA or biosensors based on sequences of nucleic acids (aptamer) designed so that they specifically enable detecting and quantifying SS'-dimethyl-gliotoxin. Furthermore proteins could be used that specifically recognize this marker (antibodies, enzymes or others) as elements of recognition in the design of specific biosensors of SS'-dimethyl-gliotoxin.

Based on the results shown in the present invention, a second aspect of the invention relates to the *in vitro* use of SS'-dimethyl-gliotoxin as marker for the diagnosis, prognosis and/or of monitoring of pathologies caused by a gliotoxin-producing fungus or its derivatives in a subject.

Since the detection of bmGT could be combined with the detection of other biological components or metabolites of fungi, a preferred embodiment of the second aspect of the invention relates to the *in vitro* use of SS'-dimethyl-gliotoxin combined with at least one biological component or metabolite of gliotoxin-producing fungi, selected from the list comprising galactomannan, (1-3)beta-D-glucan, DNA, antigens, gliotoxin, gliotoxin E, Gliotoxin G, S-methylgliotoxin, helvolic acid, sporidesmin, sterigmatocystin, aflatoxins, ochratoxins, sirodesmins, aranotin, emestryn, dithiosilvatin, fumonisins or any of their combinations. A more preferred embodiment relates to the use where the other marker (biological component or metabolite of gliotoxin-producing fungi) is gliotoxin. It is also possible to use any other metabolite or biological component of fungi not present in the list which, in combination with bmGT, is useful for the diagnosis, prognosis and/or of monitoring of pathologies caused by a gliotoxin-producing fungus or its derivatives in a subject.

Another preferred embodiment of the second aspect of the invention relates to the use where the fungus is of the genus *Aspergillus* or *Penicillium.* Another preferred embodiment relates to the use where the fungus is *Aspergillus,* preferably it is *Aspergillus fumigatus.*

Another preferred embodiment of the second aspect of the invention relates to the use where the pathology is aspergillosis. The present invention also relates to the use where the pathology is penicilliosis.

Another preferred embodiment of the second aspect of the invention relates to the use where the subject is a human. Another more preferred embodiment relates to the use where the subject is an immunodepressed subject.

Throughout the description and the claims the word "comprises" and its variants are not intended to exclude other technical characteristics, additives, components or steps. For persons skilled in the art, other objects, advantages and characteristics of the invention will be inferred in part from the description and in part from the practice of the invention. The following figures and examples are provided by way of illustration, and are not intended to limit the present invention.

### DESCRIPTION OF THE FIGURES

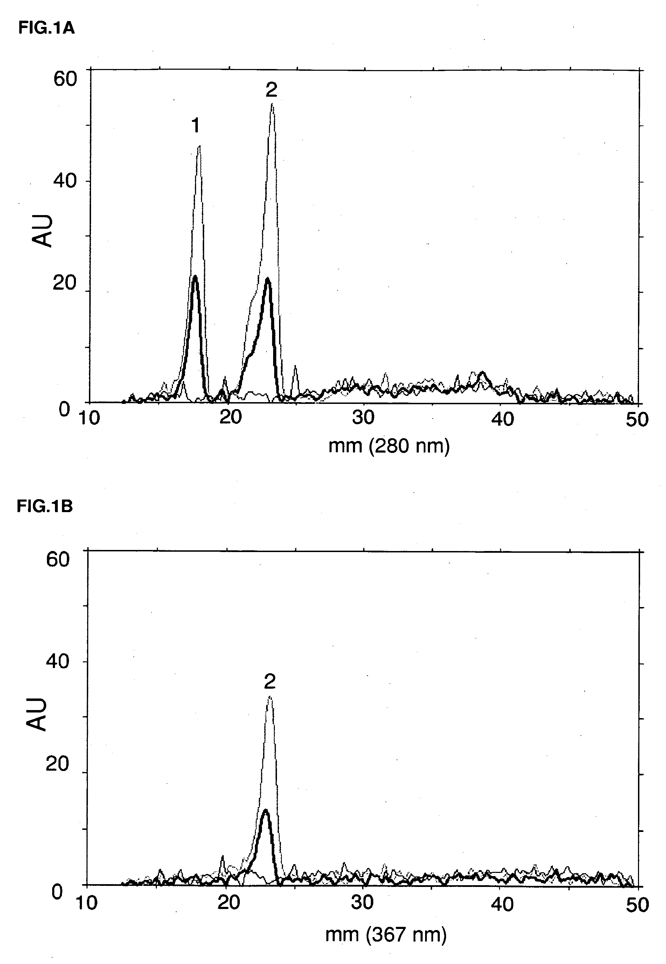

**FIG. 1****. HPTLC chromatographs of GT and BMGT after their extraction from human serums whereto known concentrations of said toxins had been added.** Quantity of each toxin added to the serum: 0.068µg; Conditions: Horizontal development using a THF/C7/ACN mixture (40:58:2 % in volume, v) during 3 min. **A,** Detection by UV-VIS at 280nm. **B,** Detection by UV-VIS (ultraviolet-visible) at 367nm. Peak 1 corresponds to bmGT and peak 2 to GT. Black curve: GT and bmGT extracted from serum. Grey curve: Pure compounds. AU, arbitrary units; mm, distance in millimetres.

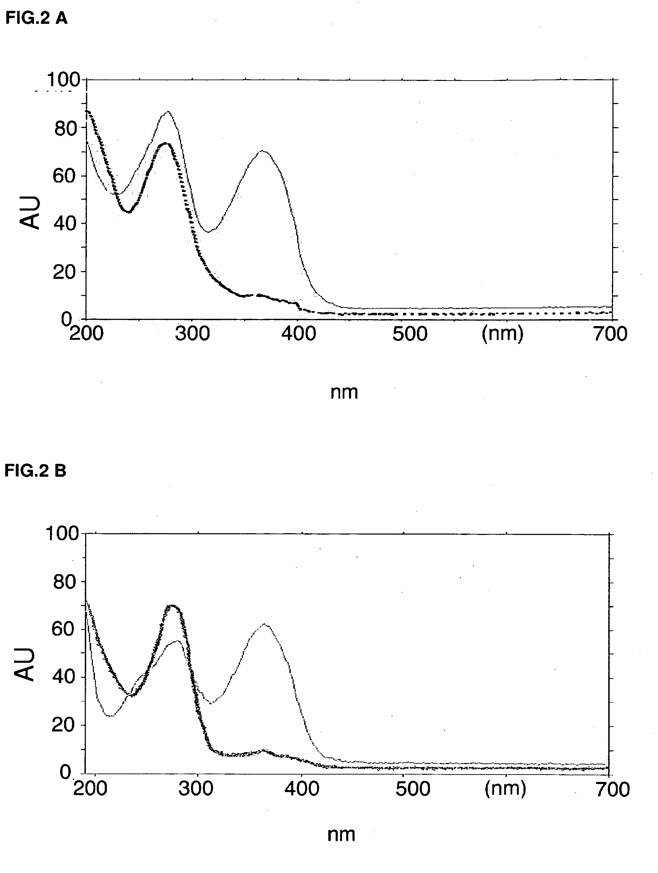

**FIG. 2****. UV-VIS spectrum of GT and bmGT after their separation using HPTLC. A, UV-VIS** spectrum corresponding to the GT peak (grey) at 22.9 mm and the bmGT peak (dotted black) at 17.6 mm after the injection of pure compounds and separation using HPTLC. **B,** UV-VIS spectrum corresponding to the GT peak (grey) and the bmGT peak (dotted black) after addition to serum, later extraction with DCM and separation by HPTLC as described in the methods. AU, arbitrary units; nm, nanometres.

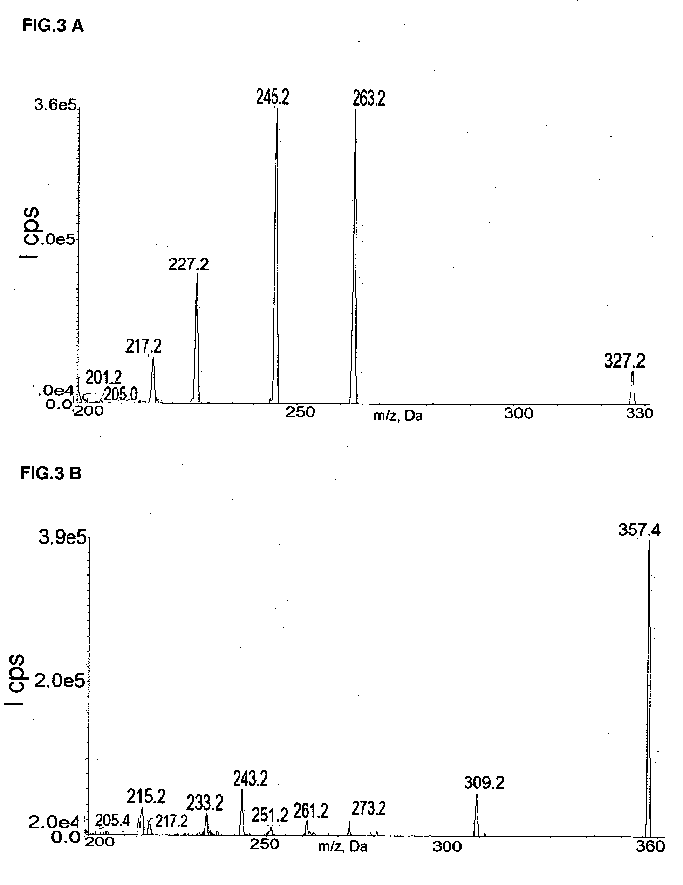

**FIG. 3****. Mass spectrums of GT and bmGT extracted from the serum.** GT and bmGT were extracted from human serum whereto known concentrations of the toxins were added and they were separated by HPTLC. Later, the compounds were extracted from the plates using a MS-interface with MeOH. **A**, MS2 mass spectrum of the molecular ion m/z 327 corresponding to the GT extracted from a HPTLC plate (peak at 22.9 mm). **B**, MS2 mass spectrum of the molecular ion m/z 357 corresponding to the bmGT extracted from a HPTLC plate (development conditions of figure 1; peak at 17.6 mm). I cps (intensity in counts per second). M/z Da (mass/charge quotient, molecular mass in Dalton).

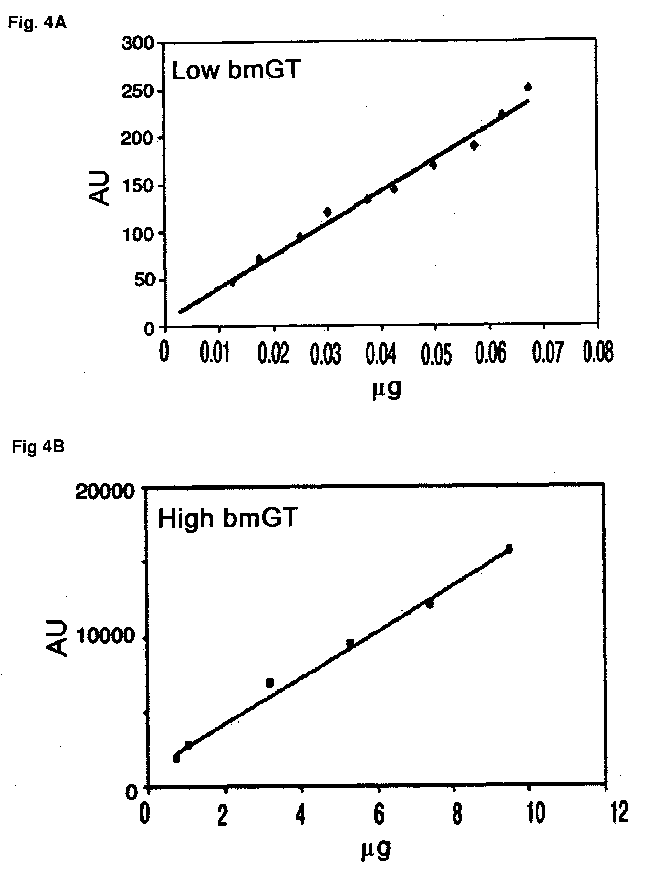

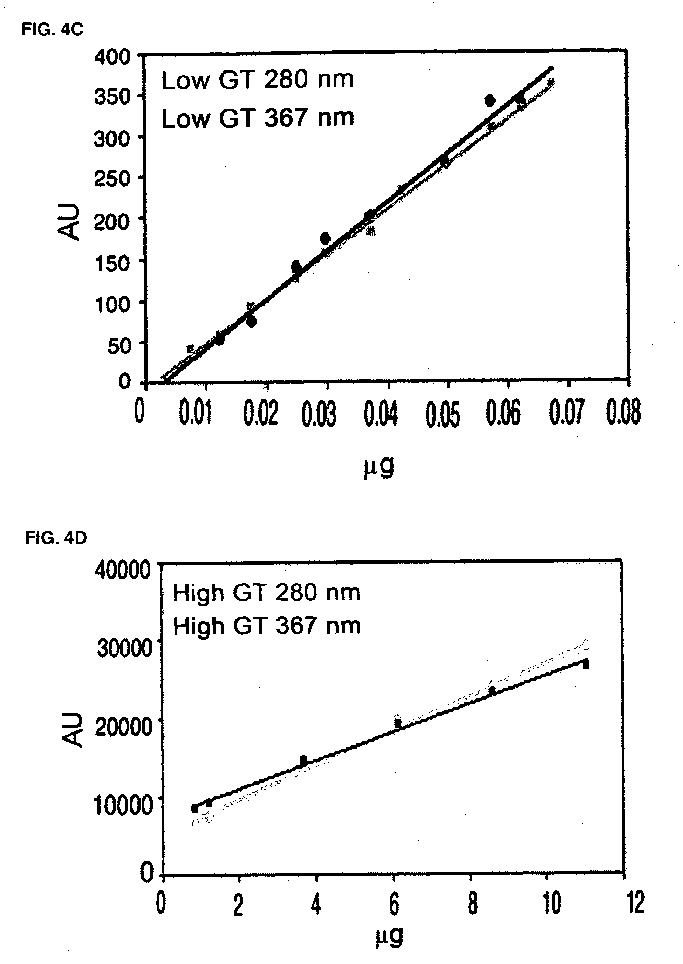

**FIG. 4****. Calibration curves of GT and bmGT in human serum. A and B**, curves of low (A) and high (B) concentrations of bmGT detected by UV absorption at 280 nm (low concentration: 3394.5 x + 7.1114, R2 = 0.982; high concentration: 1514.1x + 1238, R2 = 0.9927). **C and D**, curves of low (C) and high (D) concentrations of GT detected by UV absorption at 280 nm in grey (low concentration: 5386.4 x - 6.0911, R2 = 0.9967; high concentration: 2183.9x + 5144.3, R2 = 0.9964) and at 367 nm in black (low concentration: 5861.6 x - 17.388, R2 = 0.9868; high concentration: 1794.7x + 7362.7, R2 = 0.9936). Both toxins were added to human serum, extracted with DCM and separated by HPTLC. AU, arbitrary units; µg, micrograms).

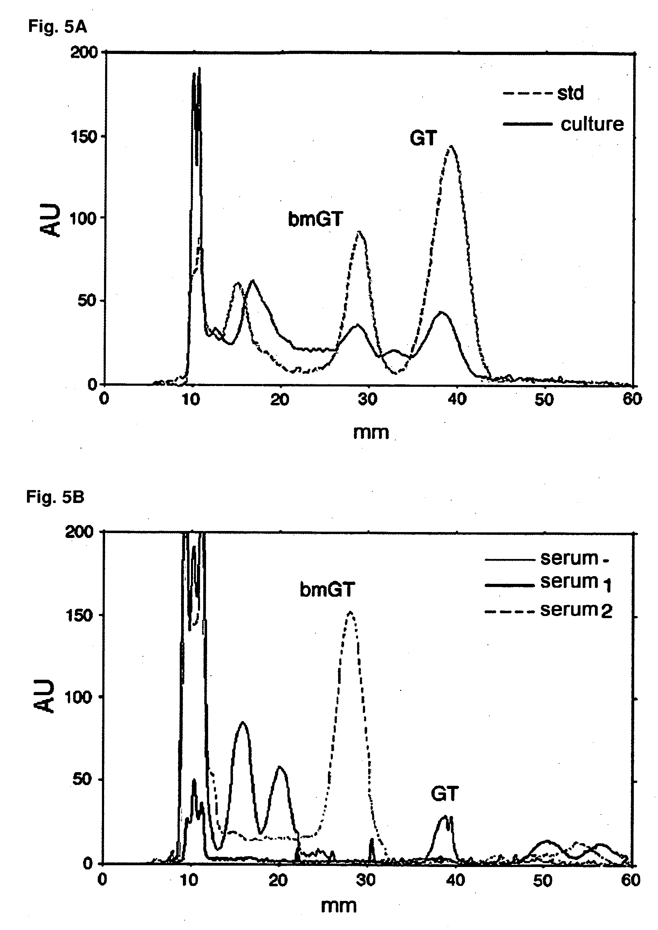

**FIG. 5****. Separation and detection of GT and bmGT in samples of *A. fumigatus* isolated from patients or in serum of patients with risk of IA. A,** HPTLC chromatogram of GT and bmGT extracted from human serum whereto known concentrations of toxins had been added (standard, std) or from a culture of a strain of *A. fumigatus* isolated from a patient (culture). **B**, HPTLC chromatogram of the products extracted from samples of human serum of patients with risk of IA (serum 1 and 2) or from healthy donors (serum -). Conditions: Horizontal development with THF/C7/ACN (40:58:2 v%) during 3 minutes. The identity of the peaks corresponding to GT and bmGT (indicated) was determined using the analysis of the UV-Visible spectrum at 280 nm and 367 nm. AU, arbitrary units; mm, distance in millimetres.

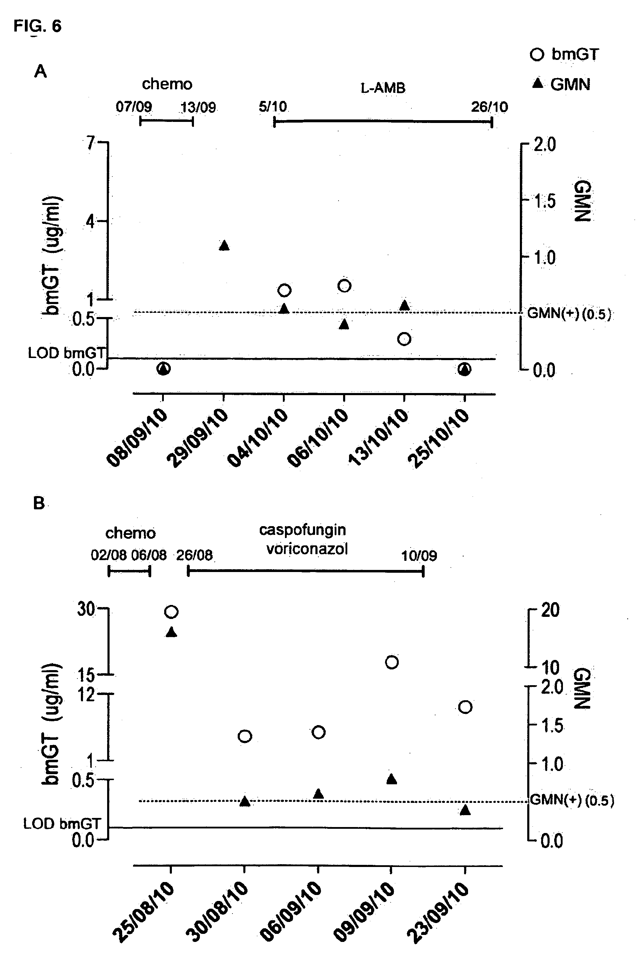

**FIG. 6****. Analysis of the presence of GT and bmGT in a sequential series of serum of patients with risk of invasive aspergillosis. A: patient with acute myeloid leukemia. B: patient with acute lymphatic leukemia.** The concentration of bmGT (left axis) is indicated by open circles and the GMN index (galactomannan; right axis) by closed triangles. The LD (limit of detection) of bmGT and positivity of GMN (0.5) are represented by continuous and broken lines, respectively. The upper horizontal bars indicate the periods of time wherein the patients had been treated with chemotherapy (A: idarubicin plus cytarabine; B: cyclophosphamide / methotrexate / cytarabine / hydrocortisone) as well as with antifungal therapy (A: liposomal amphotericin; B: caspofungin and voriconazole). Active GT was detected in no case.

### EXAMPLES

The following specific examples provided in this document serve to illustrate the nature of the present invention. These examples are only included for illustrative purposes and are not to be interpreted as limitations to the invention claimed herein. Therefore, the examples described below illustrate the invention without limiting the field of application thereof.

Below, the invention will be illustrated using assays performed by the inventors, revealing the usefulness of bmGT as it is a better marker of the presence of fungi than GT, and therefore, better marker of the diagnosis of pathologies caused by gliotoxin-producing fungi or their derivatives, among them, *Aspergillus.*

### Example 1:

### Materials and methods

### Reagents and moulds:

Gliotoxin (GT) and bis(methylthio)gliotoxin (SS'-dimethyl-gliotoxin - bmGT-) (purity: 99%) were obtained from Enzo Life Sciences. Their chemical structures are shown below:

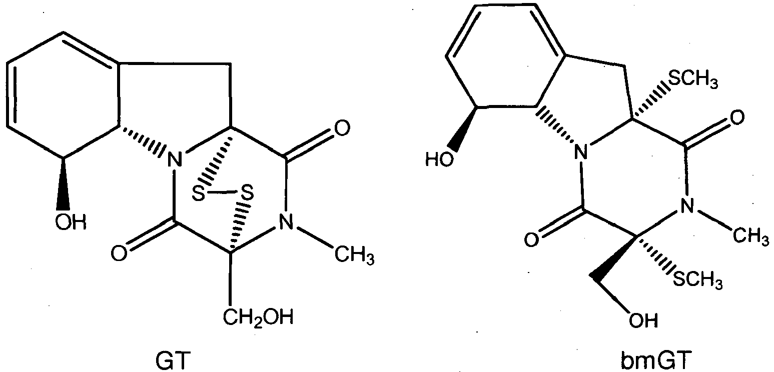

The human serum AB was from Invitrogen. The strain of *Aspergillus fumigatus* (*A. fumigatus*) B5233 (Sugui JA et al. 2007 Eukaryot Cell 6(9):1562-1569) was provided by June-Kwon-Chung (NIH / NIAID, USA).

### Human samples:

Serum, plasma and blood from healthy donors were obtained from the Blood and Tissue Bank of Aragon, Zaragoza, Spain. Bronchoalveolar lavages and serums were obtained from patients with a risk of invasive aspergillosis (IA) in the Microbiology Service of the Hospital Miguel Servet, Zaragoza. IA was classified as possible / probable or proven in accordance with EORTC / MSG guidelines for the classification of fungal infections (Ascioglu S et al. 2002 Clin Infec Dis 34:7-14).

The galactomannan index in human samples was determined routinely in the Microbiology Service (Hospital Miguel Servet, Zaragoza) using the Platelia Aspergillus® test, Bio-Rad. All protocols were supervised and approved by the Clinical Research Ethics Committee of Aragon (CEICA).

### Thin layer chromatography (HPTLC):

Plates: high performance silica gel plates (HPTLC plates, in glass of 10 cm × 10 cm; particle size 4.8 microns; pore size 60A; thick layer of 0.2 mm) were from Merck (Darmstadt, Germany) and Macherey Nagel (Düren, Germany).

### Preparation of the sample and application:

1 - Standards: GT and bmGT were dissolved in MeOH, and were applied on the plates HPTLC using a Linomat IV sample applicator (Camag, Muttenz, Switzerland), as 4 mm strips.
2 - *in vitro* addition on serum and plasma: The pure compounds or mixtures were dissolved in human serum or plasma, they were extracted with dichloromethane (DCM) (HPLC grade, Scharlau, Barcelona, Spain) (1:1 (v: v)) and were applied on the plates. They were later separated by HPTLC as described below. The efficacy of the extraction (precision and accuracy) was determined after the addition of known quantities of GT and bmGT of human serum or plasma and the later quantification of the extracted compounds.
3 - *in vitro* addition on blood: The pure compounds or mixtures thereof were dissolved in the human blood and were incubated at ambient temperature during 30 min. Later, the blood was centrifuged at 2000 revolutions per minute (rpm) during 15 minutes, the serum was recovered and GT and bmGT were extracted and analysed as described above.
4 - Mould culture filtrates: The mould culture filtrates were prepared as described above (Sugui JA et al. 2007 Eukaryot Cell 6(9):1562-1569). Briefly, the spores were suspended in RPMI 1640 culture medium (Gibco) at a density of 1x10⁵ spores / ml. After 72 hours at 37 ºC, the suspension was filtered through 0.2 µm filters (BD) and was stored at -80 ºC. GT and bmGT were extracted and analysed as described above.

### Chromatographic development:

The samples were separated using a horizontal developing chamber (Camag) by facing the plates downwards. n-heptane (C7), MeOH, tetrahydrofuran (THF) and acetonitrile (ACN) (HPLC grade, Scharlau, Barcelona, Spain) were used as solvents. The separation and quantification of GT and bmGT was performed with a mixture of THF: C7: ACN (40:58:2) (v: v: v) during 3 min.

Detection and quantification by UV spectroscopy, a TLC3 scanner was used (Camag) in UV mode whereby a UV scanning densitometry was carried out to determine GT and bmGT (wavelength (λ) = 280 and 367 nm). A linear scan with a beam of 4 mm × 0.2 mm was used in both cases. The area under the peaks was quantified and saved using Camag's software.

### Mass spectrometry (MS):

An HPTLC-MS interface from Camag was used, to extract GT and bmGT from the HPTLC plates, using MeOH as solvent.

For the identification of GT and bmGT, a MS Sciex MDS, 3200 Q TRAMPA LC / MS / MS (Applied Biosystems) system was used, equipped with an electrospray ionization source (ESI).

### Results

Although GT was originally isolated and characterized by thin layer chromatography (TLC) (Mullbacher A et al. 1985. J Gen Microbiol 131 (5):1251-1258; Mullbacher A et al. 1984 Proc Natl Acad Sci USA 81 (12):3835-3837), the typical detection methods are currently based on high performance liquid chromatography (HPLC) and mass spectrometry (MS) (Sulyok et al. 2007 Anal Bioanal Chem 389(5):1505-1523; Vishwanath V et al. 2009 Anal Bioanl Chem 395(5):1355-1372). Here we have used a method based on HPTLC to separate, detect and quantify GT and bmGT, that is more sensitive than those described in the state of the art. The main advantages of the use of HPTLC compared with HPLC are that the operations are less expensive, the faster results and the possibility of analysing several samples at the same time for comparative analysis. Furthermore, they can be executed by non-specialized laboratory staff. For example, we have been capable of analysing (detection, separation and quantification) 10 samples in less than 10 minutes, including recovery of the sample.

In first place, the optimum conditions were established for the extraction and separation of GT and bmGT. For this we have used mixtures of pure compounds or commercial human serum whereto known quantities of these mixtures have been added. The optimum separation was obtained with the mixture of solvents C7: THF: ACN (58:40:2) (FIG. 2). This elution protocol was used in all subsequent studies.

Then, the limit of detection (LOD) and quantification (LOQ) of the different techniques were determined. With this objective, mixtures of GT and bmGT and commercial human serum were made in the range of 1 to 75 ng/point of application for each toxin. The samples were applied to the HPTLC plate, the chromatograms were developed and the signal obtained from the samples was compared with the blank samples (human serum without toxins) applied at the same time. The sample which produced a signal-noise ratio of 3 was selected as LOD and of 10 as LOQ.

### UV detection:

In first place, the absorption spectrums of GT and bmGT were analysed after their elution from mixtures of pure compounds. The spectrums had two absorption maximums at 280 and 367 nm for GT, whilst only one maximum was observed at 280 nm for bmGT (FIG 1 A). It is important to highlight that these differences between the spectrums are useful for differentiating and quantifying the compounds. In order to assess if the components of the serum induced changes in the UV spectrum of GT and bmGT, the effect of the human spectrum was analysed. GT or bmGT was added to commercial human serum, and the compounds were later extracted, they were applied on the plates, they were separated and the UV spectrums were analysed. As shown in FIG 2B, the GT and bmGT spectrums were identical to those of the pure compounds (FIG 2A). By the use of UV detection, we have been capable of detecting 5 and 10 ng of GT and bmGT, respectively. The limits of detection obtained are better than with other more complex methods than those used in the present invention. The smallest quantity of GT used in methods such as HPLC-MS was 15 ng in samples of food (Sulyok M et al. Anal Bioanal Chem 389(5):1505-1523) and 20 ng in cultures of *A. fumigatus* (Kupfahl C et al. 2008 Int J Med Microbiol 298(3-4):319-327) or in samples of human serum (Lewis RE et al. 2005 Infect Immun 73(1):635-637). An important problem for this type of techniques (HPCL-MS) could be a matrix effect which causes a suppression of the signal, which reduces the sensitivity of the detection. Other methods of HPLC needed more than 45 ng of pure GT (Kupfahl C et al. 2008 Int J Med Microbiol 298(3-4):319-327; Kupfahl C et al. FEMS Yeast Res 7(6):986-992; Richard JL et al. Mycopathologia 107(2-3):145-151) added to food or biological samples. Comparison with other methods using TLC indicates that more than 100 ng / point of application were required to detect GT (Puri A et al. 2010 Biomed Chromatogr 24 (8):887-892; Wilhite S et al. 1994 Phytopathology 84(8):816-821). The method disclosed in the present invention makes it possible to detect even smaller quantities of GT and bmGT than specific bioassays that needed more than 18 ng of compound (Grovel OR et al. 2006 J Microbiol Methods 66(2):286-293). This detection method (UV) was chosen for the detection and quantification of GT and bmGT in human samples. Recently, Puri et al. (Puri A et al. 2010 Biomed Chromatogr 24 (8):887-892) described a method, using HPTLC, for the quantitative determination of GT in the serum of patients with aspergillosis but in said work the limit of detection was of 25 ng / ml of GT and the range of the sample applied 100-1000 ng/point of application. These quantities are significantly higher than the quantity detected in the present invention and therefore, the method of the invention is more sensitive and, therefore, more useful for the early detection of diseases caused by GT-producing fungi.

### Precision and accuracy of GT and bmGT detection:

To define the sensitivity and the precision of our method, we have analysed the recovery percentage of GT and bmGT from human serum and plasma, as well as the relative standard deviation (RSD) of four independent measurements performed on four different days (Table 1). For this, different quantities of GT and bmGT were added to the serum or plasma of healthy donors. Later, GT and bmGT were extracted, separated and quantified as described above.

**Table 1. Efficiency of the recovery of GT and bmGT from human serum, plasma and blood.**

| Sample | Mass¹ | % Gliotoxin (GT)² | | | % Bis-methyl-Gliotoxin (bmGT)² | | |
|---|---|---|---|---|---|---|---|
| | | mean | sd | RSD | mean | sd | RSD |
| Serum | 0.05 µg | 77.97 | 14.63 | 18.77 | 75.95 | 11.71 | 15.42 |
| | 0.5 µg | 94.34 | 7.20 | 7.63 | 96.10 | 8.19 | 8.53 |
| | 5 µg | 91.79 | 9.10 | 9.91 | 94.65 | 6.09 | 6.43 |
| Plasma | 0.05 µg | 80.09 | 6.30 | 7.87 | 85.28 | 4.22 | 4.94 |
| | 0.5 µg | 98.14 | 4.23 | 4.31 | 94.96 | 2.07 | 2.18 |
| | 5 µg | 95.99 | 5.55 | 5.78 | 96.33 | 8.18 | 8.49 |
| Blood | 5 µg | 8.44 | 0.34 | 16.08 | 73.21 | 2.31 | 12.85 |
| | 20 µg | 6.56 | 0.17 | 10.42 | 87.24 | 2.01 | 9.23 |

| | | | | | | | |
|---|---|---|---|---|---|---|---|
| ¹Mass of GT or bmGT added to 1 ml of total serum, plasma or blood. Before the extraction, the blood was centrifuged to eliminate the cellular fraction. ² The percentage (%) of recovery was calculated as the mean and standard deviation (SD) of 4 independent extractions. | | | | | | | |

As shown in table 1, the recovery of GT and bmGT from the serum and the plasma was similar, being greater than 90% in the majority of the cases. Only when low quantities of compounds were added (50 ng), was the recovery slightly less, but still around 80% in most cases. The values of RSD varied from 2 to 18%, which indicates an acceptable level of reproducibility of the method. Recovery of GT and bmGT from human blood:
In parallel, the influence of the presence of blood cells on the recovery of GT and bmGT was analysed. Unlike the serum and plasma, less than 10% of GT was recovered from the whole blood, even when large quantities of GT (20 ug) were added (Table 1). In contrast, the percentage of bmGT recovered was still very high and comparable with that of serum and plasma. The precision of this determination was as high as in the serum or plasma with RSD values of between 9-16%. Aliquots of blood and plasma from the same unit of blood were used simultaneously to perform these determinations, therefore, the only, but crucial, difference between both fluids is the presence of red and white cells in the blood. This result confirms that GT, but not bmGT, was quickly eliminated by the cells *in vitro* and, therefore, cannot be detected in the fluids of patients with IA. Despite GT being detected in the serum of infected patients, our results suggest that bmGT is better to control the presence of fungi in immunodepressed patients since the results shown here demonstrate that it is not eliminated by the patient's cells.

### Specificity of GT and bmGT detection:

Our data suggest that it is possible to selectively detect GT if the absorption is measured at 367 nm. Previous works have always used the measurements at 254 nm (Puri A et al. 2010 Biomed Chromatogr 24(8):887-892; Richard JL et al. 1989 Mycopathologia 107(2-3): 145-151). Our results indicate that the combination of measurements both at 280 and 367 nm is the most precise way to assess the presence of biologically active GT, as well as the ratio of bmGT and GT. This is very important for establishing a more precise diagnosis and a prognosis of IA, since, as indicated above, GT may be incorporated by the cells, masking its detection (Table 1) and producing false negatives in the diagnosis of IA.

One of the advantages of the method of the invention is that the presence of GT and bmGT can be assessed without the need to use MS analysis. To validate this statement, it was decided to confirm the identity of GT and bmGT by MS. After the separation of GT and bmGT from human serum by HPTLC, both samples were extracted in MeOH using a HPTLC-MS interface, in order to confirm the identity of the two peaks using MS as shown in Figure 3. The samples were directly infused in the ESI probe with an automatic syringe. A complete analysis of MS showed a more abundant ion at m / z 349 for GT and 380 for bmGT, corresponding to respective sodium adducts [M + Na]+. The molecular ion [M]+ was also found at m / z 327.2 and 357.4, in each case, corresponding to the molecular weight calculated from the molecular form of GT and bmGT.

In both cases, the molecular ion [M]+ was selected for fragmentation in Multiple Reaction Monitoring (MRM). The fragmentation of the compound with m / z 327,2 resulted in four majority fragments with m / z 263.2, 245.2, 227.2 and 217.2 with a collision energy (CE) of 50. In the case of the compound with m / z 357.4 the collision with CE of 50, gave rise to fragments of m / z 309.2, 273.2 and 261.2. The fragmentation template shown in the GT spectrum indicates a loss of 64 units m/z, which corresponds to a disulfide bridge [M-S2] +. In the case of its dimethylated analogue the lack of 47 m/z units is shown corresponding to a -SMe [M-SMe]+ group. These data confirm that these molecules correspond to GT and bmGT, respectively.

### Quantification of GT and bmGT:

Once the optimum conditions for extracting, separating and detecting GT and bmGT were established and the precision, sensitivity and specificity were determined, this method was used to quantify GT and bmGT in human serum, plasma and blood. *A priori* we did not know the quantity of GT and/or bmGT that may be present in the fluids of patients with IA. Therefore, the linearity of our detection system was analysed in a wide range of concentrations. Two calibration lines were calculated representing high and low concentrations for each compound using the addition of decreasing quantities of GT and bmGT to human serum (Figure 4).

The lines were calculated at the wavelengths at which the maximum of UV absorption had been observed for these compounds (FIG 2A). 280 nm and 367 nm in the case of GT and 280 nm in the case of bmGT. In all cases, the linearity was very good with R² values greater than 0.98.

### Detection of GT and bmGT in samples of patients:

Finally, it was analysed if the technique described was capable of detecting GT and/or bmGT in fluids of human beings with possible / probable or proven IA. The majority of the samples analysed were taken from immunodepressed patients with risk of IA (possible / probable), because the number of samples of cases of positively identified IA (autopsy) was very small.

In first place, it was analysed if we could detect GT and bmGT in a filtrate prepared from a culture of a strain of *A. fumigatus* isolated from a patient. As is shown in figure 5A, we have been capable of detecting both compounds. As in the case of this isolate, from a total of 50 isolates clinically analysed, we have been capable of finding bmGT in 95% whilst GT was detected in 70% of the cases, which suggest that the majority of the clinical isolates of *A. fumigatus* produce the marker bmGT.

Next, the samples of human serums were processed and, after elution of the HPTLC plates, the concentration of GT and/or bmGT was calculated using the extrapolation of the corresponding calibration line values. An example of the elution profile of the serum samples is shown in figure 5B. In all cases studied, the concentration of GT was considerably lower than that of bmGT, which suggest yet again that bmGT remains for a longer time in human fluids than the active compound.

**Table 2. Detection of GT and bmGT in samples of patients at risk of or with possible / probable or proven IA.**

| Sample¹ | Disease² | IA³ | Galactomann an Index⁴ | GT (µg/ml) | bmGT (µg/ml) |
|---|---|---|---|---|---|
| 1 | ALL | Possible | 0.5 | - | 5 |
| 2 | COPD | Probable | 2.06 | 2.02 | - |
| 3 | ALL | Probable | 1.8 | - | 0.46 |
| 4 | AML | Probable | 0.5 | - | 1.56 |
| 5 | AML | possible | - | - | - |
| 6 | AML | possible | - | - | 49.84 |
| 7 | ALL | possible | - | - | 3.17 |
| 8 | MS | possible | - | 0.19 | + |
| 9 | B-CLL | possible | - | - | 2.04 |
| 10 | AML | possible | - | - | 2.17 |
| 11 | AML | possible | - | - | - |
| 12 | B-CLL | possible | - | - | - |
| 13 | AML | possible | - | - | - |
| 14 | MM | possible | - | - | - |
| 15 | Ewing's sarcoma | possible | - | - | - |
| 16 | Acute leukemia | possible | - | - | - |
| 17 | Thrombocyt openia | risk | - | - | - |
| 18 | Myeloprolife ration | possible | - | - | - |
| 19 | Heart transplant | possible | - | - | - |
| 20 | ALL | probable | 7.1 | - | + |
| 21 | MG | probable | 1.4 | 0.25 | + |
| 22 | ALL | possible | - | - | - |
| 23 | Cerebral lymphoma | possible | - | - | - |
| 24 | MM | possible | - | - | 0.83 |
| 25 | AML | possible | - | - | 0.40 |
| 26 | AML | possible | - | - | - |
| 27 | Bone marrow transplant | possible | - | - | - |
| 28 | Oncology | possible | - | - | 5.05 |
| 29 | AML | possible | - | - | 0.47 |
| 30 | Heart transplant | probable | 1.22 | - | 3.52 |
| 31 | AML | possible | - | - | |
| 32 | Haematolog ical oncology | possible | - | - | 14.26 |
| 33 | MS | possible | - | - | - |
| 34 | B-cell Lymphoma | possible | - | - | - |
| 35 | Pancytopeni a. | possible | - | - | 19.99 |
| 36 | healthy | - | - | - | - |
| 37 | healthy | - | - | - | - |
| 38 | healthy | - | - | - | - |
| 39 | healthy | - | - | - | - |
| 40 | healthy | - | - | - | - |
| 41 | healthy | - | - | - | - |
| 42 | healthy | - | - | - | - |
| 43 | healthy | - | - | - | - |
| 44 | healthy | - | - | - | - |
| 45 | AML | - neutropenia⁵ | - | - | - |
| 46 | AML | - neutropenia⁵ | - | - | - |
| 47 | ALL | - neutropenia⁵ | - | - | - |
| 48 | AML | - neutropenia⁵ | - | - | - |
| 49 | AML | - neutropenia⁵ | - | - | - |
| 50 | BMT | - neutropenia⁵ | - | - | - |
| 51 | BMT | - neutropenia⁵ | | | |
| 52 | BMT | - neutropenia⁵ | | | |

| | | | | | |
|---|---|---|---|---|---|
| ¹ all samples are serum except 20 and 21 which are bronchoalveolar lavages. To obtain the serum, the blood was centrifuged at 2000 rpm for 15 minutes and the soluble fraction was collected. The bronchoalveolar lavages were filtered with a 0.2 µm filter. The extraction was performed with dichloromethane as described above. ² underlying disease to IA. ALL: Acute Lymphoblastic Leukemia AML: Acute Myeloid Leukemia; MG: Monoclonal Gammopathy; CLL: Chronic Lymphatic Leukemia; MS: Myelodysplastic Syndrome; MM: Multiple Myeloma. ³ according to the EORTC/MSG guidelines for the classification of fungal infections ⁴ Index over 0.5 is considered positive + positive sample below the quantification limit (10 ng/ml) ⁵ control samples from patients with neutropenia but without any evidence of mycological infection. Together with the samples of healthy patients, they indicate that the bmGT marker is not detected in persons without risk or with no evidence of infection. | | | | | |

As shown in table 2, the method of the invention is capable of detecting bmGT in 3 out of 4 serums of patients with probable IA (75%) and 12 of 28 with possible IA (43%). It should be highlighted that from the possible cases, galactomannan was only detected in 1 of them. GT was only detected in one of the serums with probable IA (25%) and in another with possible (3%). bmGT was detected in the two samples of bronchoalveolar lavages that were available (samples 20 and 21). It should be highlighted that we were capable of detecting bmGT in samples 6, 7, 8, 9, 10, 24, 25, 28, 29, 32 and 35 where galactomannan (the current main marker for diagnosis of IA) was not detectable, which suggests that bmGT may be an earlier marker to reveal the presence of *Aspergillus.* It should be indicated that in all possible samples where bmGT was not detected, neither was galactomannan detected nor was there any evidence of mycological infection. Two different groups have been used as controls: healthy donors and neutropenic patients (at risk of infection by aspergillus) wherein there is no evidence of infection (fever, cough, breathing difficulty, etc). GT or bmGT were not detected in any of these groups.

In the majority of the cases, the concentration of GT was much lower than the concentration of bmGT, which suggests that the prevalence of GT in the serum is more transitory than bmGT. This result confirms our hypothesis that the mean life of bmGT in the serum is longer than that of GT and is, therefore, a better diagnostic marker.

Therefore, the necessary studies have been performed to confirm the precision and sensitivity of the detection of SS'-dimethyl-gliotoxin in human serum using (line layer chromatography with UV detection). Likewise, it has been confirmed that the detection method is reliable (really detects SS'-dimethyl-gliotoxin and not other similar compounds) using mass spectrometry (LC-MS-MS). In the present invention, samples from the blood of healthy donors, neutropenic patients without evidence of possible aspergillosis or patients with probable aspergillosis have been used. SS'-dimethyl-gliotoxin has not been found in any of the samples of healthy donors or neutropenic donors without evidence of infection. Our data indicate that whilst the active form of gliotoxin is present in some of them, SS'-dimethyl-gliotoxin is present in a much more significant percentage of said samples (around 75% in probable samples-positive in galactomannan- and 43% in possible samples and it is even detected in 40% of negative samples in galactomannan (table 2). This may be explained by the fact that GT is highly active and quickly metabolized by the organism's tissues. On the other hand, bmGT is inert and is not incorporated in the tissues for which reason its presence in the organism's fluids is more stable. In fact, our data indicate that if we add GT to samples of total blood and we analyse their plasma levels (liquid fraction of the blood) after 10 minutes, 90% of GT has been lost in the blood cells (Table 1). On the other hand, only 10-15% of bmGT is lost in these conditions. This fact indicates to us that the detection of bmGT produces much less false negatives than the detection of GT. Furthermore, in some samples we are capable of detecting bmGT before galactomannan, current marker of invasive aspergillosis (Table 2). The analyses have been carried out in samples of human serum obtained from peripheral blood and in bronchoalveolar lavages (table 2). Other data which support the present invention have been generated using strains of *Aspergillus fumigatus* isolated from persons with confirmed Aspergillosis. Our data indicate that the majority of these clinical isolates are capable of producing bmGT, which supports the generality of said marker. Therefore, the results obtained serve as a base for the *in vitro* use of bmGT as described in the second aspect of the invention and, furthermore, the present invention shows a method based on the detection of bmGT for the diagnosis, prognosis and/or monitoring as described in the first aspect of the invention.
Analysis of the presence of GT and bmGT in a sequential series of serum from patients at risk of invasive aspergillosis (patient with acute myeloid leukemia and patient with acute lymphatic leukemia).

As shown in figure 6A, neither galactomannan nor bmGT were detected during the first stages of chemotherapy (idarubicin plus cytarabine). However, the level of both compounds considerably increased after treatment, when the patient has serious neutropenia. Treatment with liposomal amphotericin B liposomal (L-AMB) progressively reduced the levels of both markers. It is important to highlight that we have found more positive samples for bmGT than for galactomannan between days 04/10 and 13/10. bmGT was detected in all samples, whilst one sample was negative for galactomannan (06/10) and another was at its limit of detection (04/10). The results for the second patient (figure 6B) were similar to those of the first. bmGT and galactomannan appeared after the patient was treated with intensive chemotherapy (cyclophosphamide / methotrexate / cytarabine / hydrocortisone) and they decreased after the antifungal treatment. These two examples again illustrate that bmGT may be used as specific marker of invasive aspergillosis and that it may help to improve the current methods used in the diagnosis of this infection.

## Claims

1. A method for the *in vitro* diagnosis, prognosis and/or monitoring of pathologies caused by fungi producing gliotoxin or derivatives of gliotoxin, **characterized by** the detection of SS'-dimethyl-gliotoxin in the biological sample of a subject, wherein the fungi is a fungi of the genus *Aspergillus.*

2. The method according to claim 1, wherein the detection is carried out using the following steps:
a) separation of SS'-dimethyl-gliotoxin in the sample by chromatography;
b) detection and/or quantification of the compound separated in (a) by ultraviolet spectroscopy.

3. The method according to claim 2, wherein the separation of step (a) is carried out by thin layer chromatography (HPTLC).

4. The method according to any of claims 2 or 3, wherein the detection of step (b) is performed between 220 and 400 nm.

5. The method according to claim 4, wherein the detection of step (b) is performed at 280nm.

6. The method according to any of claims 1 to 5, wherein the fungus is *Aspergillus fumigatus.*

7. The method according to any of claims 1 to 6, wherein the pathology is aspergillosis, preferably the aspergillosis is invasive aspergillosis.

8. The method according to any of claims 1 to 7, wherein the subject is human.

9. The method according to any of claims 1 to 8, wherein the biological sample is selected from serum, plasma, blood or bronchoalveolar lavage.

10. The method according to any of claims 1 to 9, wherein it further comprises the detection of biological components or metabolites of fungi, wherein said components or metabolites are selected from the list comprising: galactomannan, (1-3)beta-D-glucan, DNA, antigens, gliotoxin, gliotoxin E, Gliotoxin G, S-methylgliotoxin, helvolic acid, sirodesmins, aranotin, emestryn, dithiosilvatin, sporidesmin, sterigmatocystin, aflatoxins, ochratoxins, fumonisins or any of their combinations.

11. The method according to claim 10, wherein gliotoxin is detected by the following steps:
a) separation of gliotoxin in the sample, by chromatography;
b) detection and/or quantification of the compound separated in (a) by ultraviolet spectroscopy.

12. The method according to claim 11, wherein the detection of step (b), for the gliotoxin, is performed between 220-400 nm, preferably at 280 and 367 nm.

13. *In vitro* use of SS'-dimethyl-gliotoxin as marker for the diagnosis, prognosis and/or monitoring of pathologies caused by fungus producing gliotoxin or derivatives of gliotoxin in a subject, preferably the pathology is invasive aspergillosis, wherein the fungi is a fungi of the genus *Aspergillus.*

14. *In vitro* use of SS'-dimethyl-gliotoxin according to claim 13, combined with at least one biological component or metabolite of gliotoxin-producing fungi, selected from the list comprising galactomannan, (1-3)beta-D-glucan, DNA, antigens, gliotoxin, gliotoxin E, Gliotoxin G, S-methylgliotoxin, helvolic acid, sporidesmin, sterigmatocystin, aflatoxins, ochratoxins, fumonisins or any of their combinations.

## Patentansprüche

1. Eine Methode für die In-vitro-Diagnostik, -Prognose und/oder - Überwachung von Pathologien, welche durch Pilz, welche Gliotoxin oder Derivative von Gliotoxin produzieren, verursacht werden. Charakterisiert durch den Nachweis von SS'-Dimethyl-Gliotoxin in der biologischen Stichprobe eines Subjekts, worin der Pilz von der Gattung Aspergillus ist.

2. Die Methode gemäß Behauptung 1, worin der Nachweis mittels der folgenden Schritte gemacht wird:
a) Separation von SS'-Dimethyl-Gliotoxin bei der Stichprobe mittels Chromatografie;
b) Erkennung und/oder Quantifizierung der Verbindung getrennt in (a) durch Ultraviolettspektroskopie

3. Die Methode gemäß Behauptung 2, worin die Trennung von Schritt (a) mittels Dünnschichtchromatografie (HPTLC) gemacht wird.

4. Die Methode gemäß irgendeiner der Behauptungen 2 und 3, worin die Erkennung von Schritt (b) zwischen 220 und 400 nm durchgeführt wird.

5. Die Methode gemäß Behauptung 4, worin die Erkennung von Schritt (b) bei 280 nm durchgeführt wird.

6. Die Methode gemäß irgendeiner der Behauptungen 1 bis 5, worin der Pilz *Aspergillus fumigatus ist.*

7. Die Methode gemäß irgendeiner der Behauptungen 1 bis 6, worin die Pathologie Aspergillosis ist, am besten ist der Aspergillosis ein invasiver Aspergillosis.

8. Die Methode gemäß irgendeiner der Behauptungen 1 bis 7 worin das Subjekt menschlich ist

9. Die Methode gemäß irgendeiner der Behauptungen 1 bis 8, worin die biologischen Proben aus Serum, Plasma, Blut oder bronchoalveolären Lavagen ausgwählt werden

10. Die Methode gemäß irgendeiner der Behauptungen 1 bis 9, worin es zusätzlich den Nachweis und die biologischen Komponenten der Stoffwechselprodukte von Pilzen beinhaltet, worin die erwähnten Komponenten oder Stoffwechselprodukte aus einer Liste ausgewählt werden, die folgendes umfasst: 5 Galactomannan, (1-3)beta-D-Glucan, DNA, Antigene, Gliotoxin, Gliotoxin E, Gliotoxin G, S-Methylgliotoxin, Helvolsäure, Sirodesmins, Aranotin, Emestryn, Dithiosilvatin, Sporidesmin, Sterigmatocystin, Aflatoxine, Ochratoxine, Fumonisin oder irgendwelche anderen Kombinationen.

11. Die Methode gemäß Behauptung 10, worin das Gliotoxin durch die folgenden Schritte nachgewiesen wird:
a) Trennung von Gliotoxin in der Stichprobe mittels Chromatographie;
b) Erkennung und/oder Quantifizierung der Verbindung getrennt in (a) durch Ultraviolettspektroskopie

12. Die Methode gemäß Behauptung 11, worin der Nachweis für Schritt (b), für das Gliotoxin, zwischen 220-400 nm durchgeführt wird, am besten zwischen 280 und 367 nm.

13. *In vitro*-Einsatz des SS'-Dimethyl-Gliotoxin als Marker für die Diagnose, Prognose und/oder Überwachung von Pathologien, verursacht durch Pilze, die Gliotoxin oder Derivative von Gliotoxin in einem Subjekt produzieren, vorzugsweise ist die Pathologie eine invasive Aspergillose, worin der Pilz ein Pilz der Gattung Aspergillus ist.

14. *In vitro*-Einsatz des SS'-Dimethyl-Gliotoxin gemäß Behauptung 13, kombiniert mit mindestens einem biologischen Komponenten oder Metabolit von Gliotoxin-produzierenden Pilzen, ausgewählt aus der Liste, die Galaktomannan, 1-3)beta-D-Glucan, DNA, Antigene, Gliotoxin, Gliotoxin E, Gliotoxin G, S-Methylgliotoxin, Helvolsäure, Sporidesmin, Sterigmatocystin, Aflatoxine, Ochratoxin, Fumonisin oder irgendwelche Ihrer Kombinationen, enthält.

## Revendications

1. Un procédé pour le diagnostic *in vitro,* le pronostic et/ou la surveillance de pathologies provoquées par des champignons produisant de la gliotoxine ou des dérivés de gliotoxine **caractérisé par** la détection de SS'-diméthyl-gliotoxine dans l'échantillon biologique prélevé chez un sujet, dans lequel les champignons sont des champignons du genre *Aspergillus.*

2. Le procédé selon la revendication 1, dans lequel la détection est réalisée en respectant les étapes suivantes :
a) séparation de la SS'-diméthyl-gliotoxine dans l'échantillon par chromatographie ;
b) détection et/ou quantification du composé séparé dans l'étape a) par spectroscopie ultraviolette.

3. Le procédé selon la revendication 2, dans lequel la détection de l'étape a) est réalisée par chromatographie en couches minces (CCMHP).

4. Le procédé selon n'importe laquelle des revendications 2 ou 3, dans lequel la détection de l'étape b) est réalisée entre 220 et 400 nm.

5. Le procédé selon la revendication 4, dans lequel la détection de l'étape b) est réalisée à 280 nm.

6. Le procédé selon n'importe laquelle des revendications 1 à 5, dans lequel le champignon est l'*Aspergillus fumigatus.*

7. Le procédé selon n'importe laquelle des revendications 1 à 6, dans lequel la pathologie est l'aspergillose, l'aspergillose étant de préférence l'aspergillose invasive.

8. Le procédé selon n'importe laquelle des revendications 1 à 7, dans lequel le sujet est un humain.

9. Le procédé selon n'importe laquelle des revendications 1 à 8, dans lequel l'échantillon biologique est sélectionné parmi sérum, plasma, sang ou lavage broncho-alvéolaire.

10. Le procédé selon n'importe laquelle des revendications 1 à 9, dans lequel est incluse en outre la détection de composants biologiques ou de métabolites de champignons, dans lequel lesdits composants ou métabolites sont sélectionnés dans la liste comprenant: galactomannane, (1-3)bêta-D-glucane, ADN, antigènes, gliotoxine, gliotoxine E, gliotoxine G, S-méthylgliotoxine, acide helvolique, sirodesmins, aranotine, emestryn, dithiosilvatine, sporidesmine, stérigmatocystine, aflatoxines, ochratoxines, fumonisines ou n'importe laquelle de leurs combinaisons.

11. Le procédé selon la revendication 10, dans lequel la gliotoxine est détectée par les étapes suivantes :
a) séparation de la gliotoxine dans l'échantillon, par chromatographie ;
b) détection et/ou quantification du composé séparé dans l'étape a) par spectroscopie ultraviolette.

12. Le procédé selon la revendication 11, dans lequel la détection de l'étape b), pour la gliotoxine, est réalisée entre 220-400 nm, de préférence à 280 et 367 nm.

13. Utilisation *in vitro* de SS'-diméthyl-gliotoxine en tant que marqueur pour le diagnostic, le pronostic et/ou la surveillance de pathologies provoquées par un champignon produisant de la gliotoxine ou des dérivés de gliotoxine chez un sujet, la pathologie étant de préférence l'aspergillose invasive, dans laquelle les champignons sont des champignons du genre *Aspergillus.*

14. Utilisation *in vitro* de SS'-diméthyl-gliotoxine selon la revendication 13, associée à au moins un composant biologique ou métabolite de champignons produisant de la gliotoxine, sélectionné dans la liste comprenant : galactomannane, (1-3)bêta-D-glucane, ADN, antigènes, gliotoxine, gliotoxine E, gliotoxine G, S-méthylgliotoxine, acide helvolique, sporidesmine, stérigmatocystine, aflatoxines, ochratoxines, fumonisines ou n'importe laquelle de leurs combinaisons.
